# EUROPEAN PATENT SPECIFICATION

(11) **EP 2 474 558 B1**
(45) Date of publication and mention of the grant of the patent: **15.04.2015**
(21) Application number: 10813369.5
(22) Date of filing: 27.09.2010
(51) Int. Cl.: C07K 19/00, C07K 16/12, C12N 15/62, C12N 15/63, C12P 21/02, A61K 38/16, A61K 47/48, A61P 31/04, C07K 14/245

(54) **NEW ANTIBIOTIC CONTAINING SIMULACRUM ANTIBODY, PREPARATION METHOD AND APPLICATION THEREOF**
NEUES ANTIBIOTIKUM MIT EINEM SIMULAKRUMSANTIKÖRPER, HERSTELLUNGSVERFAHREN DAFÜR UND ANWENDUNG DAVON
NOUVEL ANTIBIOTIQUE CONTENANT UN ANTICORPS SIMULACRE, PROCÉDÉ DE PRÉPARATION ET APPLICATION DE CELUI-CI

(30) Priority: 02.09.2009 CN 200910092128
(43) Date of publication of application: 11.07.2012
(73) Proprietor: Protein Design Lab, Ltd., Beijing 100095 (CN)
(72) Inventor: QIU, Xiaoqing, Chengdu, Sichuan 610041 (CN)
(74) Representative: Grünecker Patent- und Rechtsanwälte PartG mbB
(86) International application number: PCT/CN2010/077351
(87) International publication number: WO 2011/026447

(56) References cited:
- WO-A1-2007/083175
- WO-A1-2007/083175
- WO-A2-2006/109192
- CN-A- 1 766 110
- CN-A- 101 215 568
- CN-A- 101 633 699
- MUTHUKKARUPPAN V R ET AL: "Monoclonal antibodies against Salmonella porins: Generation and characterization", IMMUNOLOGY LETTERS, ELSEVIER BV, NL, vol. 33, no. 2, 1 July 1992 (1992-07-01), pages 201-206, XP023888448, ISSN: 0165-2478, DOI: 10.1016/0165-2478(92)90047-R [retrieved on 1992-07-01]
- LIAO YING ET AL.: 'Bactericidal peptide targeted against penicillin/methicillin-resistant Staphylococcus aureus-an engineered multidomain protein machine.' J SICHUAN UNIV (MED SCI EDI). vol. 34, no. 4, 2003, pages 605 - 609, XP008159286
- XIAO-QING QIU ET AL.: 'A novel engineered peptide, a narrow-spectrum antibiotic, is effective against vancomycin-resistant Enterococcus faecalis.' ANTIMICROBIAL AGENTS AND CHEMOTHERAPY vol. 49, no. 3, March 2005, pages 1184 - 1189, XP002462086
- XIAO-QING QIU ET AL.: 'An engineered multidomain bactericidal peptide as a model for targeted antibiotics against specific bacteria.' NATURE BIOTECHNOLOGY vol. 21, no. 12, December 2003, pages 1480 - 1485, XP003010122
- Anonymous: "Chain L, Bactericidal Antibody Against Neisseria Meningitidis - Protein - NCBI", , 20 September 2013 (2013-09-20), XP55140105, Retrieved from the Internet: URL:http://www.ncbi.nlm.nih.gov/protein/55 42523 [retrieved on 2014-09-15]
- Anonymous: "Chain H, Bactericidal Antibody Against Neisseria Meningitidis - Protein - NCBI", , 20 September 2013 (2013-09-20), XP55140112, Retrieved from the Internet: URL:http://www.ncbi.nlm.nih.gov/protein/55 42524 [retrieved on 2014-09-15]

### SEQUENCE LISTING

<110> Protein Design Lab, Ltd.
<120> A novel antibiotic comprising an antibody mimetic, preparation methods and uses thereof
<130> EP80795HV163pau
<140> 10 813 369.5
   <141> 2010-09-27
<150> PCT/CN2010/077351
   <151> 2010-09-27
<150> 200910092128.4
   <151> 2009-09-02
<160> 22
<170> PatentIn version 3.3
<210> 1
   <211> 81
   <212> DNA
   <213> Artificial sequence
<220>
   <223> The nucleotide sequence of antibody mimetic AM1
<400> 1
   tcttattggc tgcattggat taaacagaga cctggtcagg gactgtggat cggatctcag      60
   tccacgcatg tgccgagaac c                                                81
<210> 2
   <211> 27
   <212> PRT
   <213> Artificial sequence
<220>
   <223> The nucleotide sequence of antibody mimetic AM2
<400> 2
   Ser Tyr Trp Leu His Trp Ile Lys Gln Arg Pro Gly Gln Gly Leu Trp
   1               5                   10                  15
   Ile Gly Ser Gln Ser Thr His Val Pro Arg Thr
               20                  25
<210> 3
   <211> 81
   <212> DNA
   <213> Artificial sequence
<220>
   <223> The amino acid sequence of antibody mimetic AM2.
<400> 3
   tcttattggc tgcattggat taaacagaga cctggtcagg gactgtggat cggaaccaga      60
   ccggtgcata cgtcccagtc t 81
<210> 4
   <211> 27
   <212> PRT
   <213> Artificial sequence
<220>
   <223> The amino acid sequence of antibody mimetic AM2.
<400> 4
   Ser Tyr Trp Leu His Trp Ile Lys Gln Arg Pro Gly Gln Gly Leu Trp
   1               5                   10                  15
   Ile Gly Thr Arg Pro Val His Thr Ser Gln Ser
               20                  25
<210> 5
   <211> 1959
   <212> DNA
   <213> Artificial sequence
<220>
   <223> The nucleotide sequence of antibiotic PMC-AM1
<400> 5
   atgtctgacc ctgtacgtat tacaaatccc ggtgcagaat cgctggggta tgattcagat     60
   ggccatgaaa ttatggccgt tgatatttat gtaaaccctc cacgtgtcga tgtctttcat    120
   ggtaccccgc ctgcatggag ttccttcggg aacaaaacca tctggggcgg aaacgagtgg    180
   gttgatgatt ccccaacccg aagtgatatc gaaaaaaggg acaaggaaat cacagcgtac    240
   aaaaacacgc tcagcgcgca gcagaaagag aatgagaata agcgtactga agccggaaaa    300
   cgcctctctg cggcgattgc tgcaagggaa aaagatgaaa acacactgaa aacactccgt    360
   gccggaaacg cagatgccgc tgatattaca cgacaggagt tcagactcct gcaggcagag    420
   ctgagagaat acggattccg tactgaaatc gccggatatg acgccctccg gctgcataca    480
   gagagccgga tgctgtttgc tgatgctgat tctcttcgta tatctccccg ggaggccagg    540
   tcgttaatcg aacaggctga aaaacggcag aaggatgcgc agaacgcaga caagaaggcc    600
   gctgatatgc ttgctgaata cgagcgcaga aaaggtattc tggacacccg gttgtcagag    660
   ctggaaaaaa atggcggggc agcccttgcc gttcttgatg cacaacaggc ccgtctgctc    720
   gggcagcaga cacggaatga cagggccatt tcagaggccc ggaataaact cagttcagtg    780
   acggaatcgc ttaacacggc ccgtaatgca ttaaccagag ctgaacaaca gctgacgcaa    840
   cagaaaaaca cgcctgacgg caaaacgata gtttcccctg aaaaattccc ggggcgttca    900
   tcaacaaatc attctattgt tgtgagcggt gatccgagat ttgccggtac gataaaaatc    960
   acaaccagcg cagtcatcga taaccgtgca aacctgaatt atcttctgag ccattccggt   1020
   ctggactata aacgcaatat tctgaatgac cggaatccgg tggtgacaga ggatgtggaa   1080
   ggtgacaaga aaatttataa tgctgaagtt gctgaatggg ataagttacg gcaaagattg   1140
   cttgatgcca gaaataaaat cacctctgct gaatctgcgg taaattcggc gagaaataac   1200
   ctcagtgcca gaacaaatga gcaaaagcat gcaaatgacg ctcttaatgc cctgttgaag   1260
   gaaaaagaga atatacgtaa ccagctttcc ggcatcaatc agaagatagc ggaagagaaa   1320
   agaaaacagg atgaactgaa ggcaacgaaa gacgcaatta atttcacaac agagttcctg   1380
   aaatcagttt cagaaaaata tggtgcaaaa gctgagcagt tagccagaga gatggccggg   1440
   caggctaaag ggaagaaaat acgtaatgtt gaagaggcat taaaaacgta tgaaaagtac   1500
   cgggctgaca ttaacaaaaa aattaatgca aaagatcgtg cagcgattgc cgcagccctt   1560
   gagtctgtga agctgtctga tatatcgtct aatctgaaca gattcagtcg gggactggga   1620
   tatgcaggaa aatttacaag tcttgctgac tggatcactg agtttggtaa ggctgtccgg   1680
   acagagaact ggcgtcctct ttttgttaaa acagaaacca tcatagcagg caatgccgca   1740
   acggctcttg tggcactggt cttcagtatt cttaccggaa gcgctttagg cattatcggg   1800
   tatggtttac tgatggctgt caccggtgcg ctgattgatg aatcgcttgt ggaaaaagcg   1860
   aataagttct ggggtatttc ttattggctg cattggatta aacagagacc tggtcaggga   1920
   ctgtggatcg gatctcagtc cacgcatgtg ccgagaacc                          1959
<210> 6
   <211> 652
   <212> PRT
   <213> Artificial sequence
<220>
   <223> The amino acid sequence of antibiotic PMC-AM1
<400> 6
   Ser Asp Pro Val Arg Ile Thr Asn Pro Gly Ala Glu Ser Leu Gly Tyr
   1               5                   10                  15
   Asp Ser Asp Gly His Glu Ile Met Ala Val Asp Ile Tyr Val Asn Pro
               20                  25                  30
   Pro Arg Val Asp Val Phe His Gly Thr Pro Pro Ala Trp Ser Ser Phe
           35                  40                  45
   Gly Asn Lys Thr Ile Trp Gly Gly Asn Glu Trp Val Asp Asp Ser Pro
       50                  55                  60
   Thr Arg Ser Asp Ile Glu Lys Arg Asp Lys Glu Ile Thr Ala Tyr Lys
   65                  70                  75                  80
   Asn Thr Leu Ser Ala Gln Gln Lys Glu Asn Glu Asn Lys Arg Thr Glu
                   85                  90                  95
   Ala Gly Lys Arg Leu Ser Ala Ala Ile Ala Ala Arg Glu Lys Asp Glu
   100 105 110
   Asn Thr Leu Lys Thr Leu Arg Ala Gly Asn Ala Asp Ala Ala Asp Ile
   115 120 125
   Thr Arg Gln Glu Phe Arg Leu Leu Gln Ala Glu Leu Arg Glu Tyr Gly
   130 135 140
   Phe Arg Thr Glu Ile Ala Gly Tyr Asp Ala Leu Arg Leu His Thr Glu
   145 150 155 160
   Ser Arg Met Leu Phe Ala Asp Ala Asp Ser Leu Arg Ile Ser Pro Arg
   165 170 175
   Glu Ala Arg Ser Leu Ile Glu Gln Ala Glu Lys Arg Gln Lys Asp Ala
   180 185 190
   Gln Asn Ala Asp Lys Lys Ala Ala Asp Met Leu Ala Glu Tyr Glu Arg
   195 200 205
   Arg Lys Gly Ile Leu Asp Thr Arg Leu Ser Glu Leu Glu Lys Asn Gly
   210 215 220
   Gly Ala Ala Leu Ala Val Leu Asp Ala Gln Gln Ala Arg Leu Leu Gly
   225 230 235 240
   Gln Gln Thr Arg Asn Asp Arg Ala Ile Ser Glu Ala Arg Asn Lys Leu
   245 250 255
   Ser Ser Val Thr Glu Ser Leu Asn Thr Ala Arg Asn Ala Leu Thr Arg
   260 265 270
   Ala Glu Gln Gln Leu Thr Gln Gln Lys Asn Thr Pro Asp Gly Lys Thr
   275 280 285
   Ile Val Ser Pro Glu Lys Phe Pro Gly Arg Ser Ser Thr Asn His Ser
   290 295 300
   Ile Val Val Ser Gly Asp Pro Arg Phe Ala Gly Thr Ile Lys Ile Thr
   305 310 315 320
   Thr Ser Ala Val Ile Asp Asn Arg Ala Asn Leu Asn Tyr Leu Leu Ser
   325 330 335
   His Ser Gly Leu Asp Tyr Lys Arg Asn Ile Leu Asn Asp Arg Asn Pro
   340 345 350
   Val Val Thr Glu Asp Val Glu Gly Asp Lys Lys Ile Tyr Asn Ala Glu
   355 360 365
   Val Ala Glu Trp Asp Lys Leu Arg Gln Arg Leu Leu Asp Ala Arg Asn
   370 375 380
   Lys Ile Thr Ser Ala Glu Ser Ala Val Asn Ser Ala Arg Asn Asn Leu
   385 390 395 400
   Ser Ala Arg Thr Asn Glu Gln Lys His Ala Asn Asp Ala Leu Asn Ala
   405 410 415
   Leu Leu Lys Glu Lys Glu Asn Ile Arg Asn Gln Leu Ser Gly Ile Asn
   420 425 430
   Gln Lys Ile Ala Glu Glu Lys Arg Lys Gln Asp Glu Leu Lys Ala Thr
               435                 440                 445
   Lys Asp Ala Ile Asn Phe Thr Thr Glu Phe Leu Lys Ser Val Ser Glu
       450                 455                 460
   Lys Tyr Gly Ala Lys Ala Glu Gln Leu Ala Arg Glu Met Ala Gly Gln
   465                 470                 475                 480
   Ala Lys Gly Lys Lys Ile Arg Asn Val Glu Glu Ala Leu Lys Thr Tyr
                   485                 490                 495
   Glu Lys Tyr Arg Ala Asp Ile Asn Lys Lys Ile Asn Ala Lys Asp Arg
               500                 505                 510
   Ala Ala Ile Ala Ala Ala Leu Glu Ser Val Lys Leu Ser Asp Ile Ser
           515                 520                 525
   Ser Asn Leu Asn Arg Phe Ser Arg Gly Leu Gly Tyr Ala Gly Lys Phe
       530                 535                 540
   Thr Ser Leu Ala Asp Trp Ile Thr Glu Phe Gly Lys Ala Val Arg Thr
   545                 550                 555                 560
   Glu Asn Trp Arg Pro Leu Phe Val Lys Thr Glu Thr Ile Ile Ala Gly
                   565                 570                 575
   Asn Ala Ala Thr Ala Leu Val Ala Leu Val Phe Ser Ile Leu Thr Gly
               580                 585                 590
   Ser Ala Leu Gly Ile Ile Gly Tyr Gly Leu Leu Met Ala Val Thr Gly
           595                 600                 605
   Ala Leu Ile Asp Glu Ser Leu Val Glu Lys Ala Asn Lys Phe Trp Gly
       610                 615                 620
   Ile Ser Tyr Trp Leu His Trp Ile Lys Gln Arg Pro Gly Gln Gly Leu
   625                 630                 635                 640
   Trp Ile Gly Ser Gln Ser Thr His Val Pro Arg  Thr
                   645                 650
<210> 7
   <211> 1959
   <212> DNA
   <213> Artificial sequence
<220>
   <223> The nucleotide sequence of antibiotic PMC-SA2
<400> 7
   atgtctgacc ctgtacgtat tacaaatccc ggtgcagaat cgctggggta tgattcagat 60
   ggccatgaaa ttatggccgt tgatatttat gtaaaccctc cacgtgtcga tgtctttcat 120
   ggtaccccgc ctgcatggag ttccttcggg aacaaaacca tctggggcgg aaacgagtgg 180
   gttgatgatt ccccaacccg aagtgatatc gaaaaaaggg acaaggaaat cacagcgtac 240
   aaaaacacgc tcagcgcgca gcagaaagag aatgagaata agcgtactga agccggaaaa 300
   cgcctctctg cggcgattgc tgcaagggaa aaagatgaaa acacactgaa aacactccgt 360
   gccggaaacg cagatgccgc tgatattaca cgacaggagt tcagactcct gcaggcagag    420
   ctgagagaat acggattccg tactgaaatc gccggatatg acgccctccg gctgcataca    480
   gagagccgga tgctgtttgc tgatgctgat tctcttcgta tatctccccg ggaggccagg    540
   tcgttaatcg aacaggctga aaaacggcag aaggatgcgc agaacgcaga caagaaggcc    600
   gctgatatgc ttgctgaata cgagcgcaga aaaggtattc tggacacccg gttgtcagag    660
   ctggaaaaaa atggcggggc agcccttgcc gttcttgatg cacaacaggc ccgtctgctc    720
   gggcagcaga cacggaatga cagggccatt tcagaggccc ggaataaact cagttcagtg    780
   acggaatcgc ttaacacggc ccgtaatgca ttaaccagag ctgaacaaca gctgacgcaa    840
   cagaaaaaca cgcctgacgg caaaacgata gtttcccctg aaaaattccc ggggcgttca    900
   tcaacaaatc attctattgt tgtgagcggt gatccgagat ttgccggtac gataaaaatc    960
   acaaccagcg cagtcatcga taaccgtgca aacctgaatt atcttctgag ccattccggt   1020
   ctggactata aacgcaatat tctgaatgac cggaatccgg tggtgacaga ggatgtggaa   1080
   ggtgacaaga aaatttataa tgctgaagtt gctgaatggg ataagttacg gcaaagattg   1140
   cttgatgcca gaaataaaat cacctctgct gaatctgcgg taaattcggc gagaaataac   1200
   ctcagtgcca gaacaaatga gcaaaagcat gcaaatgacg ctcttaatgc cctgttgaag   1260
   gaaaaagaga atatacgtaa ccagctttcc ggcatcaatc agaagatagc ggaagagaaa   1320
   agaaaacagg atgaactgaa ggcaacgaaa gacgcaatta atttcacaac agagttcctg   1380
   aaatcagttt cagaaaaata tggtgcaaaa gctgagcagt tagccagaga gatggccggg   1440
   caggctaaag ggaagaaaat acgtaatgtt gaagaggcat taaaaacgta tgaaaagtac   1500
   cgggctgaca ttaacaaaaa aattaatgca aaagatcgtg cagcgattgc cgcagccctt   1560
   gagtctgtga agctgtctga tatatcgtct aatctgaaca gattcagtcg gggactggga    1620
   tatgcaggaa aatttacaag tcttgctgac tggatcactg agtttggtaa ggctgtccgg   1680
   acagagaact ggcgtcctct tttgttaaaa acagaaacca tcatagcagg caatgccgca   1740
   acggctcttg tggcactggt cttcagtatt cttaccggaa gcgctttagg cattatcggg   1800
   tatggtttac tgatggctgt caccggtgcg ctgattgatg aatcgcttgt ggaaaaagcg   1860
   aataagttct ggggtatttc ttattggctg cattggatta aacagagacc tggtcaggga   1920
   ctgtggatcg gaaccagacc ggtgcatacg tcccagtct                          1959
<210> 8
   <211> 652
   <212> PRT
   <213> Artificial sequence
<220>
   <223> The nucleotide sequence of antibiotic PMC-SA2
<400> 8
   Ser Asp Pro Val Arg Ile Thr Asn Pro Gly Ala Glu Ser Leu Gly Tyr
   1               5                   10                  15
   Asp Ser Asp Gly His Glu Ile Met Ala Val Asp Ile Tyr Val Asn Pro
               20                  25                  30
   Pro Arg Val Asp Val Phe His Gly Thr Pro Pro Ala Trp Ser Ser Phe
           35                  40                  45
   Gly Asn Lys Thr Ile Trp Gly Gly Asn Glu Trp Val Asp Asp Ser Pro
       50                  55                  60
   Thr Arg Ser Asp Ile Glu Lys Arg Asp Lys Glu Ile Thr Ala Tyr Lys
   65                  70                  75                  80
   Asn Thr Leu Ser Ala Gln Gln Lys Glu Asn Glu Asn Lys Arg Thr Glu
                   85                  90                  95
   Ala Gly Lys Arg Leu Ser Ala Ala Ile Ala Ala Arg Glu Lys Asp Glu
               100                 105                 110
   Asn Thr Leu Lys Thr Leu Arg Ala Gly Asn Ala Asp Ala Ala Asp Ile
           115                 120                 125
   Thr Arg Gln Glu Phe Arg Leu Leu Gln Ala Glu Leu Arg Glu Tyr Gly
       130                 135                 140
   Phe Arg Thr Glu Ile Ala Gly Tyr Asp Ala Leu Arg Leu His Thr Glu
   145                 150                 155                 160
   Ser Arg Met Leu Phe Ala Asp Ala Asp Ser Leu Arg Ile Ser Pro Arg
                   165                 170                 175
   Glu Ala Arg Ser Leu Ile Glu Gln Ala Glu Lys Arg Gln Lys Asp Ala
               180                 185                 190
   Gln Asn Ala Asp Lys Lys Ala Ala Asp Met Leu Ala Glu Tyr Glu Arg
           195                 200                 205
   Arg Lys Gly Ile Leu Asp Thr Arg Leu Ser Glu Leu Glu Lys Asn Gly
       210                 215                 220
   Gly Ala Ala Leu Ala Val Leu Asp Ala Gln Gln Ala Arg Leu Leu Gly
   225                 230                 235                 240
   Gln Gln Thr Arg Asn Asp Arg Ala Ile Ser Glu Ala Arg Asn Lys Leu
                   245                 250                 255
   Ser Ser Val Thr Glu Ser Leu Asn Thr Ala Arg Asn Ala Leu Thr Arg
               260                 265                 270
   Ala Glu Gln Gln Leu Thr Gln Gln Lys Asn Thr Pro Asp Gly Lys Thr
           275                 280                 285
   Ile Val Ser Pro Glu Lys Phe Pro Gly Arg Ser Ser Thr Asn His Ser
       290                 295                 300
   Ile Val Val Ser Gly Asp Pro Arg Phe Ala Gly Thr Ile Lys Ile Thr
   305                 310                 315                 320
   Thr Ser Ala Val Ile Asp Asn Arg Ala Asn Leu Asn Tyr Leu Leu Ser
                   325                 330                 335
   His Ser Gly Leu Asp Tyr Lys Arg Asn Ile Leu Asn Asp Arg Asn Pro
               340                 345                 350
   Val Val Thr Glu Asp Val Glu Gly Asp Lys Lys Ile Tyr Asn Ala Glu
           355                 360                 365
   Val Ala Glu Trp Asp Lys Leu Arg Gln Arg Leu Leu Asp Ala Arg Asn
       370                 375                 380
   Lys Ile Thr Ser Ala Glu Ser Ala Val Asn Ser Ala Arg Asn Asn Leu
   385                 390                 395                 400
   Ser Ala Arg Thr Asn Glu Gln Lys His Ala Asn Asp Ala Leu Asn Ala
                   405                 410                 415
   Leu Leu Lys Glu Lys Glu Asn Ile Arg Asn Gln Leu Ser Gly Ile Asn
               420                 425                 430
   Gln Lys Ile Ala Glu Glu Lys Arg Lys Gln Asp Glu Leu Lys Ala Thr
           435                 440                 445
   Lys Asp Ala Ile Asn Phe Thr Thr Glu Phe Leu Lys Ser Val Ser Glu
       450                 455                 460
   Lys Tyr Gly Ala Lys Ala Glu Gln Leu Ala Arg Glu Met Ala Gly Gln
   465                 470                 475                 480
   Ala Lys Gly Lys Lys Ile Arg Asn Val Glu Glu Ala Leu Lys Thr Tyr
                   485                 490                 495
   Glu Lys Tyr Arg Ala Asp Ile Asn Lys Lys Ile Asn Ala Lys Asp Arg
               500                 505                 510
   Ala Ala Ile Ala Ala Ala Leu Glu Ser Val Lys Leu Ser Asp Ile Ser
           515                 520                 525
   Ser Asn Leu Asn Arg Phe Ser Arg Gly Leu Gly Tyr Ala Gly Lys Phe
       530                 535                 540
   Thr Ser Leu Ala Asp Trp Ile Thr Glu Phe Gly Lys Ala Val Arg Thr
   545                 550                 555                 560
   Glu Asn Trp Arg Pro Leu Phe Val Lys Thr Glu Thr Ile Ile Ala Gly
                   565                 570                 575
   Asn Ala Ala Thr Ala Leu Val Ala Leu Val Phe Ser Ile Leu Thr Gly
               580                 585                 590
   Ser Ala Leu Gly Ile Ile Gly Tyr Gly Leu Leu Met Ala Val Thr Gly
           595                 600                 605
   Ala Leu Ile Asp Glu Ser Leu Val Glu Lys Ala Asn Lys Phe Trp Gly
       610                 615                 620
   Ile Ser Tyr Trp Leu His Trp Ile Lys Gln Arg Pro Gly Gln Gly Leu
   625                 630                 635                 640
   Trp Ile Gly Thr Arg Pro Val His Thr Ser Gln Ser
                   645                 650
<210> 9
   <211> 69
   <212> DNA
   <213> Artificial sequence
<220>
   <223> Artificial sequence of constructing mutation plasmid pBHC-PorA1, 5'-3'
<400> 9
   gcgaataagt tctggggtat ttcttattgg ctgcattgga ttaaacagta aataaaatat 60
   aagacaggc 69
<210> 10
   <211> 69
   <212> DNA
   <213> Artificial sequence
<220>
   <223> Artificial sequence of constructing mutation plasmid pBHC-PorA1, 3'-5'
<400> 10
   gcctgtctta tattttattt actgtttaat ccaatgcagc caataagaaa taccccagaa 60
   cttattcgc 69
<210> 11
   <211> 69
   <212> DNA
   <213> Artificial sequence
<220>
   <223> Artificial sequence of constructing mutation plasmid pBHC-PorA1, 5'-3'
<400> 11
   tggctgcatt ggattaaaca gagacctggt cagggactgt ggatcggata aataaaatat 60
   aagacaggc 69
<210> 12
   <211> 69
   <212> DNA
   <213> Artificial sequence
<220>
   <223> Artificial sequence of constructing mutation plasmid pBHC-PorA1, 3'-5'
<400> 12
   gcctgtctta tattttattt atccgatcca cagtccctga ccaggtctct gtttaatcca 60
   atgcagcca 69
<210> 13
   <211> 69
   <212> DNA
   <213> Artificial sequence
<220>
   <223> Artificial sequence of constructing mutation plasmid pBHC-PorA1, 3'-5'
<400> 13
   ggtcagggac tgtggatcgg atctcagtcc acgcatgtgc cgagaaccta aataaaatat 60
   aagacaggc 69
<210> 14
   <211> 69
   <212> DNA
   <213> Artificial sequence
<220>
   <223> Artificial sequence of constructing mutation plasmid pBHC-PorA1, 3'-5'
<400> 14
   gcctgtctta tattttattt aggttctcgg cacatgcgtg gactgagatc cgatccacag 60
   tccctgacc 69
<210> 15
   <211> 69
   <212> DNA
   <213> Artificial sequence
<220>
   <223> Artificial sequence of constructing mutation plasmid pBHC-PorA2, 5'-3'
<400> 15
   gcgaataagt tctggggtat ttcttattgg ctgcattgga ttaaacagta aataaaatat 60
   aagacaggc 69
<210> 16
   <211> 69
   <212> DNA
   <213> Artificial sequence
<220>
   <223> Artificial sequence of constructing mutation plasmid pBHC-PorA2, 3'-5'
<400> 16
   gcctgtctta tattttattt actgtttaat ccaatgcagc caataagaaa taccccagaa 60
   cttattcgc 69
<210> 17
   <211> 69
   <212> DNA
   <213> Artificial sequence
<220>
   <223> Artificial sequence of constructing mutation plasmid pBHC-PorA2, 5'-3'
<400> 17
   tggctgcatt ggattaaaca gagacctggt cagggactgt ggatcggata aataaaatat 60
   aagacaggc 69
<210> 18
   <211> 69
   <212> DNA
   <213> Artificial sequence
<220>
   <223> Artificial sequence of constructing mutation plasmid pBHC-PorA2, 3'-5'
<400> 18
   gcctgtctta tattttattt atccgatcca cagtccctga ccaggtctct gtttaatcca 60
   atgcagcca 69
<210> 19
   <211> 69
   <212> DNA
   <213> Artificial sequence
<220>
   <223> Artificial sequence of constructing mutation plasmid pBHC-PorA2, 5'-3'
<400> 19
   ggtcagggac tgtggatcgg aaccagaccg gtgcatacgt cccagtctta aataaaatat 60
   aagacaggc 69
<210> 20
   <211> 69
   <212> DNA
   <213> Artificial sequence
<220>
   <223> Artificial sequence of constructing mutation plasmid pBHC-PorA2, 3'-5'
<400> 20
   gcctgtctta tattttattt aagactggga cgtatgcacc ggtctggttc cgatccacag      60
   tccctgacc                                                              69
<210> 21
   <211> 225
   <212> PRT
   <213> Mus musculus
<300>
   <308> PUBMED Accession 2MPA_H
   <309> 1999-06-09
<400> 21
   Glu Val Asn Leu Gln Gln Ser Gly Thr Val Leu Ala Arg Pro Gly Ala
   1               5                   10                  15
   Ser Val Arg Met Ser Cys Lys Ala Ser Gly Tyr Ser Phe Thr Ser Tyr
               20                  25                  30
   Trp Leu His Trp Ile Lys Gln Arg Pro Gly Gln Gly Leu Glu Trp Ile
           35                  40                  45
   Gly Gly Ile Tyr Pro Gly Asn Arg Asp Thr Arg Tyr Thr Gln Arg Phe
       50                  55                  60
   Lys Asp Lys Ala Lys Leu Thr Ala Val Thr Ser Ala Asn Thr Ala Tyr
   65                  70                  75                  80
   Met Glu Leu Ser Ser Leu Thr Asn Glu Asp Ser Ala Val Tyr Tyr Cys
                   85                  90                  95
   Ser Ile Ile Tyr Phe Asp Tyr Ala Asp Phe Ile Met Asp Tyr Trp Gly
               100                 105                 110
   Gln Gly Thr Thr Val Thr Val Ser Ser Ala Lys Thr Thr Ala Pro Ser
           115                 120                 125
   Val Tyr Pro Leu Ala Pro Val Cys Gly Asp Thr Thr Gly Ser Ser Val
       130                 135                 140
   Thr Leu Gly Cys Leu Val Lys Gly Tyr Phe Pro Glu Pro Val Thr Leu
   145                 150                 155                 160
   Thr Trp Asn Ser Gly Ser Leu Ser Ser Gly Val His Thr Phe Pro Ala
                   165                 170                 175
   Val Leu Gln Ser Asp Leu Tyr Thr Leu Ser Ser Ser Val Thr Val Thr
               180                 185                 190
   Ser Ser Thr Trp Pro Ser Gln Ser Ile Thr Cys Asn Val Ala His Pro
           195                 200                 205
   Ala Ser Ser Thr Lys Val Asp Lys Lys Ile Glu Pro Arg Gly Pro Thr
       210                 215                 220
   Ile
   225
<210> 22
   <211> 219
   <212> PRT
   <213> Mus musculus
<300>
   <308> PUBMED Accession 2MPA_L
   <309> 1999-06-09
<400> 22
   Asp Ile Val Met Thr Gln Thr Pro Leu Ser Leu Pro Val Ser Leu Gly
   1               5                   10                  15
   Asp Lys Ala Ser Ile Ser Cys Arg Ser Ser Gln Ala Leu Val His Ser
               20                  25                  30
   Asn Gly Asn Thr Tyr Leu His Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Phe Phe Cys Ser Gln Ser
                   85                  90                  95
   Thr His Val Pro Arg Thr Phe Gly Gly Gly Thr Lys Leu Glu Ile Lys
               100                 105                 110
   Arg Ala Asp Ala Ala Pro Thr Val Ser Ile Phe Pro Pro Ser Ser Glu
           115                 120                 125
   Gln Leu Thr Ser Gly Gly Ala Ser Val Val Cys Phe Leu Asn Asn Phe
       130                 135                 140
   Tyr Pro Lys Asp Ile Asn Val Lys Trp Lys Ile Asp Gly Ser Glu Arg
   145                 150                 155                 160
   Gln Asn Gly Val Leu Asn Ser Trp Thr Asp Gln Asp Ser Lys Asp Ser
                   165                 170                 175
   Thr Tyr Ser Met Ser Ser Thr Leu Thr Leu Thr Lys Asp Glu Tyr Glu
               180                 185                 190
   Arg His Asn Ser Tyr Thr Cys Glu Ala Thr His Lys Thr Ser Thr Ser
           195                 200                 205
   Pro Ile Val Lys Ser Phe Asn Arg Asn Glu Cys
       210                 215

## Description

### FIELD OF THE INVENTION

The present invention belongs to the domain of biology and medicine, and especially relates to a novel antibiotic comprising an antibody mimetic, its preparation methods and uses thereof.

### RELATED ART

Since Penicillin and other antibiotics were brought into use in 1944, Diplococcus meningitides, and other life-threatening pathogenic bacteria such as *Staphylocnccus aureus, Streptococcus pneunioniae, Pseudonaonas aeruginosa*, and *Neisseria meningitidis* have acquired drug resistance. According to relative reports published by United States Disease Control Center (CDC) in recent years, these antibiotics would be likely to lose effectiveness completely in 10 or 20 years.

The present antibiotics kill pathogenic bacteria by restraining synthesis of cell wall, restraining or interfering the pathway of bacterial nucleic acid and protein metabolism and synthesis. However, these antibiosis methods are more likely to lead to drug resistance resulted by bacteria mutation. Therefore scientists are dedicated to develop novel antibiotics. One of the comparatively promising directions is to imitate the inter-killing mechanism among homogeneous heterologous strains so as to develop novel antibiotics. In Nature, a number of bacterial toxins can directly form ion channels on bacteria cytomembrane to kill bacteria. The typical specimen is a kind of bacterial toxin secreted by *E.col,* colicin. One of which is Colicin la, found in 1952 by Jacob. After efforts of generations, the transmembrane spatial structure of Colicin la when ion channels open as well as shut on the artificial lipide bimolecular film (Qiu et al, Major transmembrane movement associated with Colicin la channel gating. J. Gen. Physiology, 107: 313-328 (1996)) was disclosed in 1996. This established theoretical laid the basis for designing and preparing novel antibiotics on molecular level.

As mentioned above, colicin is a kind of ideal ion channel antibiotic model, but wild-type colicin can only act on homogeneous heterologous strains. We must change the targeting of the colicin so that they are capable of acting on other pathogenic bacteria. Porin, a kind of pore protein existing on the outer membrane of bacteria, mitochondria or chloroplast, allows bigger molecules to pass through. Porin also has a higher immunogenicity, and can induce high level expression of monoclonal antibody in host cells. It should be an ideal development direction for antibody research, if we can design an antibody mimetic with better recognizing ability to change the targeting of the colicin. by using the antibody specific for porins on outer membran of bacteria as the antetype of the antibody mimetic.

Muthukkaruppan et al., Immunol. Lett. 33 (1992), p. 201-6 discloses monoclonal antibodies against Salmonella porins and studies their potential protective effects against Salmonella infection.

WO 2007/083175 discloses a polypeptide comprising an antibody or antibody mimetic and a porin, wherein the antibody is specific for Epstein-Barr virus gp350/220 envelope glycoprotein and its use in virus associated cancer. WO 2007/083175 is apparently from the same inventor as the present application.

### SUMMARY OF THE INVENTION

To overcome the above technical defects and make up a deficiency in the art, the present invention provides a novel antibiotic. Its antibacterial ability is a thousand-fold more powerful than regular, antibiotics. Due to its unique action mechanism, drug resistance resulted in mutation can hardly be acquired by pathogenic bacteria. And the antibiotic will not hurt normal human cells when it kills pathogenic bacteria.

Embodiments of the invention are
1. An antibiotic comprising an antibody mimetic and a colicin, or comprising an antibody mimetic and a channel-forming domain of a colicin, the antibody mimetic covalently bonded to the carboxyl end of the polypeptide of the colicin or the channel-forming domain of the colicin, wherein the colicin is selected from the group consisting of colicin E1, la, lb, A, B, N; wherein said antibody mimetic being yielded by fusing two complementarity determining regions (CDRs), V_{H}CDR₁, and V_{L}CDR₃ through a cognate framework region (V_{H}FR₂) of an immunoglobulin; wherein the immunoglobulin specifically recognizes bacterium porins.
2. The antibiotic of embodiment 1, wherein the bacterium is *Neisseria meningitides,* the porin is PorA in the outer membrane of *Neisseria meningitidis* cells.
3. The antibiotic of embodiment 2, wherein the immunoglobulin consist of covalent bonded polypeptide of SEQ ID NO: 21 and a polypeptide of SEQ ID NO: 22.
4. The antibiotic of embodiment 1 or embodiment 2 or embodiment 3, wherein the colicin is la.
5. A peptide molecule comprising an antibody mimetic and a colicin, or comprising an antibody mimetic and a channel-forming domain of a colicin, the antibody mimetic covalently bonded to the carboxyl end of the polypeptide of the colicin or the channel-forming domain of the colicin, wherein the colicin is selected from the group consisting of colicin E1, la, lb, A, B, N; wherein said antibody mimetic being yielded by fusing two complementarity determining regions (CDRs), V_{H}CDR₁, and V_{L}CDR₃ through a cognate framework region (V_{H}FR₂) of an immunoglobulin; wherein the immunoglobulin specifically recognizes bacterium porins.
6. One of the fusion peptide molecules of embodiment 5, its amino acid sequence sets forth in SEQ ID NO.6.
7. A nucleic acid molecule encoding a fusion peptide molecule embodied in embodiment 5 or embodiment 6.
8. The nucleic acid molecule of embodiment 7, its nucleotide sequence sets forth in SEQ ID NO.5.
9. A recombinant plasmid containing a nucleic acid molecule of embodiments 7 or 8.
10. A preparation method of any one of the antibiotics embodied in embodiments 1-4, one of the recombinant plasmids of embodiment 9 is transfected into an expression system, and the antibiotic is separated and purified from expressed polypeptide.
11. The antibiotic of any one of embodiments 1-4 for use in preparing an antibacterial medicament.
12. The antibiotic for use according to embodiment 11, wherein the antibacterial medicament is used for killing *Neisseria meningitidis,* vancomycin-resistant *Enterococcus faecalis,* methicillin-*resistant Staphylococcus aureus or multidrug-resisfance Pseudomonas aeruginosa.*

A novel antibiotic comprising an antibody mimetic and a colicin,
or comprising an antibody mimetic and a channel-forming domain of a colicin,
the antibody mimetic covalently bonded to the carboxyl end of the polypeptide of the colicin or the channel-forming domain of the colicin, wherein the colicin is selected from a group consisting of colicin E1, la, 1b, A, B, N; wherein said antibody mimetic being yielded by fusing two complementarity determining regions (CDRs), V_{H}CDR₁ and V_{L}CDR₃through a cognate framework region (V_{H}FR₂) of an immunoglobulin; wherein the immunoglobulin specifically recognizes bacterium porins.

Wherein the bacterium is *Neisseria meningitides*, the porin is PorA in the outer membrane of *Neisseria meningitidis* cells.

Wherein the immunoglobulin consist of two covalent bonded polypeptides with PUBMED Accession 2MPA_H (SEQ ID No:21) and PUBMED Accession 2MPA_L (SEQ ID No:22)

Wherein the colicin is Ia.

A peptide molecule, comprising an antibody mimetic and a colicin,
or comprising an antibody mimetic and a channel-forming domain of a colicin,
the antibody mimetic covalently bonded to the carboxyl end of the polypeptide of the colicin or the channel-forming domain of the colicin, wherein the colicin is selected from a group consisting of colicin E1, Ia, Ib, A, B, N; wherein said antibody mimetic being yielded by fusing two complementarity determining regions (CDRs), V_{H}CDR₁ and V_{L} CDR3 through a cognate framework region (V_{H}FR₂) of an immunoglobulin; wherein the immunoglobulin specifically recognizes bacterium porins.

One of the foresaid peptide molecules, its amino acid sequence set forth in SEQ ID NO.6.

A nucleic acid molecule encoding any one of foresaid peptide molecules.

The nucleic acid molecule may have the nucleotide sequence set forth in SEQ ID NO.5.

A recombinant plasmids containing any one of foresaid nucleic acid molecules.

A preparation method of any one of foresaid novel antibiotics, one of foresaid recombinant plasmids is transfected into an expression system, and the novel antibiotic is separated and purified from expressed polypeptide.

Any one of foresaid novel antibiotics may be used for preparing antibacterial medicament.

The antibacterial medicament may be used for killing *Neisseria meningilidis*, vancomycin-resistant *Enterococcus faecalis,* methicillin-resistant *Staphylococcus aureus* or multidrug-resistance *Pseudomonas aeruginosa*.

The novel antibiotics of this invention are based on the foundation of colicin's characteristic of forming ion channels on the target pathogen membrane, which causes the pathogen to leak out its content and die. The targeting structure is an antibody mimetic with some domains of an immunoglobulin specifically recognizing porin protein on target pathogen. The antibody mimetic comprises two complementarity determining regions (CDRs), V_{H}CDR₁ and V_{L}CDR₃, and a cognate framework region (V_{H}FR₂) of an immunoglobulin; the three regions covalently form a linear peptide molecule as V_{H}CDR1- V_{H}FR2-V_{L}CDR₃ from amino end to carboxyl end. It is well known that the active regions of an immunoglobulin for recognition reaction are called complementary determining region that has only about several to a dozen of amino acids. The antibody mimetic in the novel antibiotic, compared with a nature antibody molecule or any present artificial reconstructed antibody such as seFv and Fab, has smaller molecular weight, nicer tissue penetration and simpler structure without most parts of frame structure and Fc fragment of a nature antibody. So it is be able to reduce immunoreaction in patients and easily guides colicin contained in the novel antibiotic to infected tissues and identifies the pathogenic bacteria. In clinical application, the novel antibiotic is directed to membrane of the target pathogenic bacteria by antibody mimetic contained, and the colicin contained forms ion channels on bacteria cytomembrane of the target bacteria and kills the target bacteria by leading to leak-out of its cytoplasm. The antibacterial ability of the novel antibiotic also applies to bacterial strains with drug resistance. The recognition sites are unique antigenic properties on bacterial surface, and not located on human cytomembrane, so the novel antibiotic is safe for human. Compared to other antibiotics that easily cause drug resistance, the antibiotic in this invention kills the pathogenic bacteria not by the porin but by the colicin aching on biomembrane of the pathogenic bacteria and forming ion channels to make the cell leaking out the cytoplasm to die. The antibody mimetic for targeting just needs to guide the colicin to the pathogenic bacteria. Bacteria's drug resistance is normally acquired by changing porin's structure and creating barrier for antibiotic's entry. The antibiotic in this invention only requires porin's antibody recognition sites for purpose of killing the pathogenic bacteria. The novel antibiotics identify the sites of porin on the membrane of bacteria, but the colicin of the novel antibiotic binds on other sites and forms ion channels to make the bacteria to leak out to die. The action sites are not the porin. So the pathogenic bacteria is not likely to acquire drug resistance to the novel antibiotic by mutating, evoluting, throwing away or changing the structure of porin which is necessary for survival. According to this inventive concept, the novel antibiotic has many variants due to the diversity of porin on bacterium surface and the diversity of the immunoglobulin recognizing the porins.

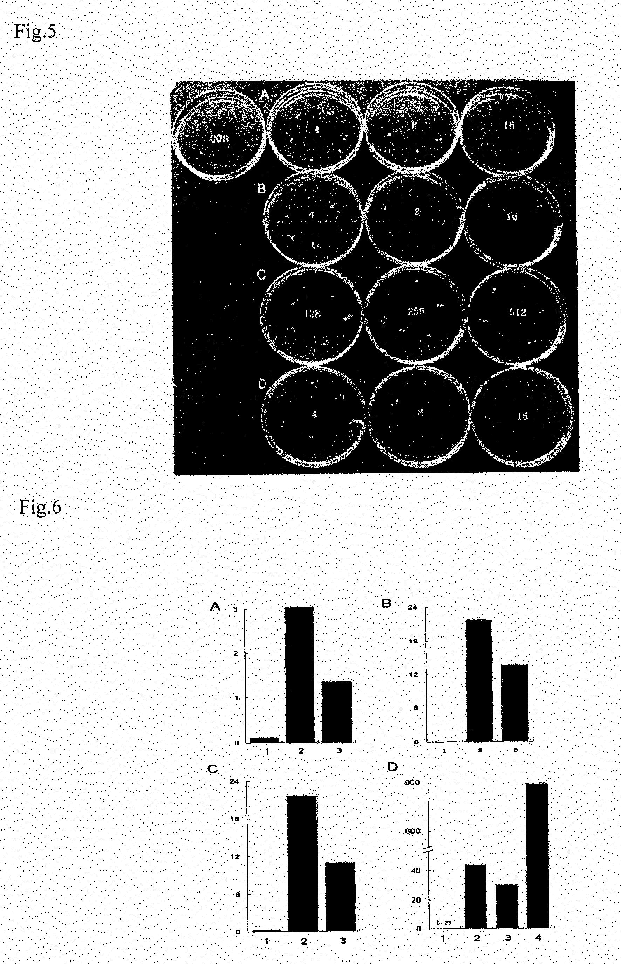

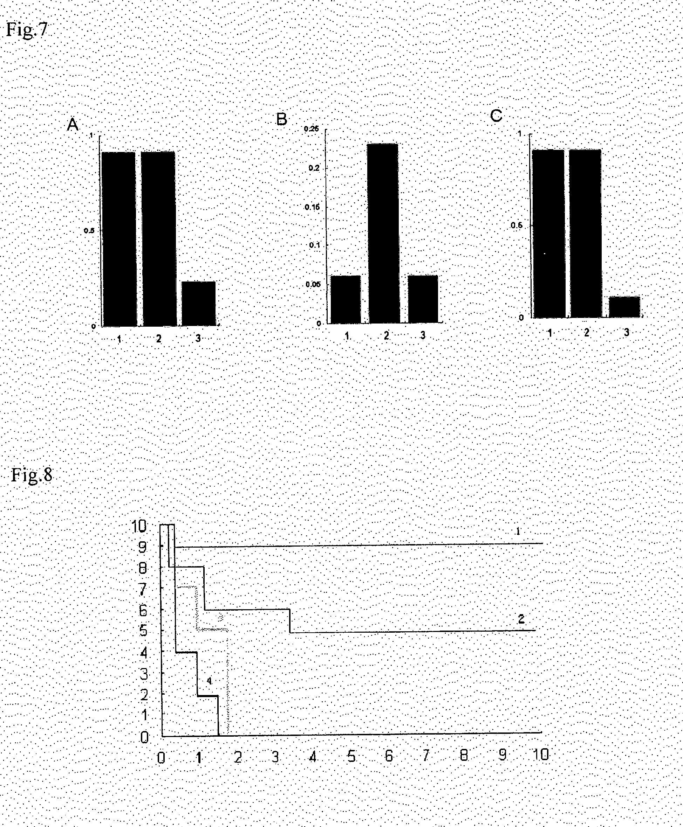

Since the meningitis caused by *Neisseria meningitidis* severe threatens infants and children's health, the drug resistance of *Neisseria meningitidis* to common medicine is very serious. The dosage is higher and higher in order to inhibit the bacteria efficiently, which seriously endangers the health of patients. Therefore, on the basis of foresaid inventive concept of this invention, the inventor reconstructed and obtained an antibody mimetic of an antibody which was specific for the porin A of *Neisseria meningitidis.* The heavy chain peptide of the antibody had an accession number: 2MPA_H at PubMed Home. The light chain peptide had an accession number: 2MPA_L at PubMed Home. The antibody mimetic, of which the amino acid sequence set forth in Seq ID No.2, was connected on the C-terminus of the Colicin Ia's peptide to constitute a novel antibiotic PMC-AM1 with amino acid sequence set forth in Seq ID No.6. As survival curve of mice shown in Fig.7, the survival rate of the mice injected with fatal dose of *Neisseria meningilides* in PMC-AM1 group was 90% in 8 days. It showed that the antibacterial activity and protective effect in vivo of the novel antibiotics are superior to current normal antibiotics such as penicillin and gentamicin. Meanwhile a comparative test on bactericidal effect was set to compare the minimum inhibitory concentration (MIC value) of PMC-AM1, ceftazidime and ampicillin for *Neisseria meningitidis.* As shown in Fig.6A, the MIC value of PMC-AM1 was 0.11nMol, ceftazidime was 3.02nMol and ampicillin was 1.35Mol. This result indicated that the bactericidal ability of PMC-AM1 is significantly more powerful than antibiotics currently used for inhibiting *Neisseria meningitidis.*

In present invention, some experiments were set to test the bactericidal effect of the novel antibiotic PMC-AM1 on other pathogens with serious drug-resistance. The result showed the PMC-AM1 had extremely stronger antibacterial ability on multi-drug resistant *Pseudomonas aeruginosa,* vancomycin-resistant *Enterococcus faecalis,* methicillin-resistant *Staphylococcus aureus,* as shown in Fig.5, its antibacterial ability on multi-drug resistant *Pseudomonas aeruginosa,* was 127 to 3800 times stronger than that of ceftazidime, levofloxacin, gentamicin etc. And as shown in Fig. 6B and Fig. 6C, PMC-AM1 has obvious inhibition effect on vancomycin-resistant *Enterococcus faecalis,* methicillin-resistant *Staphylococcus aureus.*

The novel antibiotic of this invention can be used to prepare antibacterial drugs, especially for *Neisseria meningitidis, Pseudomonas aeruginosa,* vancomycin-resistant *Enterococcus faecalis* or methicillin-resistant *Staphylococcus aureus.*

The nucleotide sequences encoding the peptides of the novel antibiotics can be cloned into the expression vector to construct recombinant plasmids, which express fusion protein in host cell. The novel antibiotic protein is isolated from expressed fusion protein.

According to degeneracy of nucleotide codons, the nucleotide sencoding antibiotic of the invention is adjustable. One of skill in the art would be able to adjust the nucleotide sequence according to the host cells' preference to the nucleotide codon. As long as the encoded polypeptide has no change, the nucleotide sequences are still in the scope of inventive concept of this invention.

### BRIEF DESCRIPTION OF THE DRAWINGS

Fig.1 shows the structure of recombinant plasmid comprising the gene of antibody mimetic and gene of Colicin Ia, referred to herein as pBHC-PorA1.
   The peptide of the antibody mimetic bonded to the C-terminus of Colicin Ia, and the amino acid sequence of the antibody mimetic is set forth in Seq ID No.2.
Fig.2 shows the structure of the recombinant plasmid comprising the gene of antibody mimetic and gene of Colicin Ia, referred to herein as pBHC-PorA2.
   The peptide of antibody mimetic is linked to the C-terminus of Ia's peptide, and the amino acid sequence of the antibody mimetic set forth in Seq ID No.4. In the antibody mimetic, a V_{H}CDR1 and a reversed V_{L}CDR₃ are connected through a cognate framework region (V_{H}FR₂).
Fig.3 illustrates the construction of the novel antibiotic.
   In which, T and R are signal recognition domains on the N-terminus of a Colicin Ia. The channel forming, a structure domain being capable of forming ion channels, is situated at C-terminus of the Colicin Ia. The AM is an antibody mimetic.
Fig.4 shows the result of experiment for inhibiting ability of the novel antibiotic PMC-AM1 to *Diplococcus intrucellularis.*
   In the curve, from left to right shows the control, 5µg/ml Ampicillin, 5µg/ml PMC-AM2, 5µg/ml PMC-AM1, 10µg/ml PMC-AM1.
   The horizontal ordinate shows growth time of bacteria, the unit is hours; the longitudinal ordinate shows optical density of the bacteria medium at 600 nm, indicating the quantity of bacteria growth.
Fig.5 shows the minimum inhibitory concentration value (MIC) of the novel antibiotic tested by Agar dilution method.
   The plates shows the MIC of the drugs to multi-drug resistant *Pseudomonas aeruginosa:* Con was blank control, (A) the MIC of ceftazidime was 16 µg / ml, (B) the MIC of levofloxacin was 8 µg / ml, (C) the MIC of gentamicin was greater than 512 µg / ml, (D) the MIC of PMC-AM1 was 8 µg / ml.
Fig.6 shows the comparison experiment of minimum inhibitory concentration of the novel antibiotic of this invention with the commonly used antibiotics to methicillin-resistant *Staphylococcus aureus* (ATCC BAA-42), vancomycin-resistant *Enterococcus faecalis* (ATCC 700802), multi-drug resistant *Pseudomonas aeruginosa* (isolated by West China Hospital, No.13578) and *Nesseria meningitidis* (No.29332 of bacteria Preservation Center in China) .
   In which, the longitudinal ordinate shows minimum inhibitory concentration (nMol);
   A shows the result of *Nesseria meningitidi:* (1) PMC-AM1, MIC = 0.11 nMol, (2) ceftazidime, MIC = 3.02 nMol, (3) ampicillin, MIC = 1.35 nMol;
   B shows the result of vancomycin-resistant *Enterococcus faecalis* (1) PMC-AM1, MIC = 0.23 nMol, (2) vancomycin, MIC = 21.54 nMol, (3) ampicillin, MIC = 10.78 nMol :
   C shows the result for methicillin-resistant *Staphylococcus aureus*:(1) PMC-AM1, MIC = 0.06 nMol, (2) ampicillin, MIC = 21.55 nMol, (3) oxacillin, MIC =14.1 nMol;
   D shows the result for multi-drug resistant *Pseudomonas aeruginosa*: (1) PMC-AM1, MIC=0.91nMol, (2) levofloxacin, MIC=43.2 nMol, (3) ceftazidime, MIC=29.3 nMol, (4) gentamicin, MIC>889.4 nmol.
Fig.7 shows survival curve of comparison of inhibition effective of the novel antibiotic, the wild-type colicin and the polypeptide anti-*Staphylococcal aureus* disclosed in China patent ZL 01128836.1 on methicillin-resistant *Staphylococcus aureus* (ATCC BAA-42), vancomycin-resistant *Enterococcus faecalis* (ATCC 700802), and multi-drug resistant *Pseudomonas aeruginosa* (isolated by West China Hospital, No.13578).
   In which, longitudinal ordinate shows minimum inhibitory concentration(nMol);
   A shows the result for vancomycin-resistant *Enterococcus faecalis:* (1) the polypeptide anti-*Staphylococcus aureus*, MIC = 0.91nMol, (2) the wild-type Colicin Ia, MIC =0.91 nMol, (3) PMC-AM1, MIC = 0.23 nmol;
   B shows the result for methicillin-resistant *Staphylococcus aureus*: (1) the polypeptide anti-*Staphylococcus aureus*, MIC = 0.06nMol, (2) the wild-type Colicin Ia, MIC =0.23 nMol, (3) PMC-AM1, MIC = 0.06 nMol.
   C shows the result for multidrug resistance *Pseudomonas aeruginosa :* (1) the polypeptide *anti-Staphylococcus aureus,* MIC = 0.91 nMol, (2) the wild-type Colicin Ia, MIC =0.91 nMol, (3) PMC-AM1,M1C=0.23 nMol.
Fig.8 shows survival curves, which were the result of in vivo experiment of the novel antibiotics to protect animals infected by *Nesseria meningitidis.*
   In which, horizontal ordinate shows the survival time of mice, days; Longitudinal ordinate shows the number of animal survival. 1) PMC-AM1; 2) Gen was gentamicin; 3) PEN was penicillin; 4) Con. was blank control. The injection concentration of all test drugs is by 1.5mg/kg (drug's weight/ mouse's weight).

### EMBODIMENTS

The invention is further illustrated by the following embodiments as well as the drawings.

### Embodiment 1: Construction of plasmids expressing the novel antibiotics and preparation of the novel antibiotics

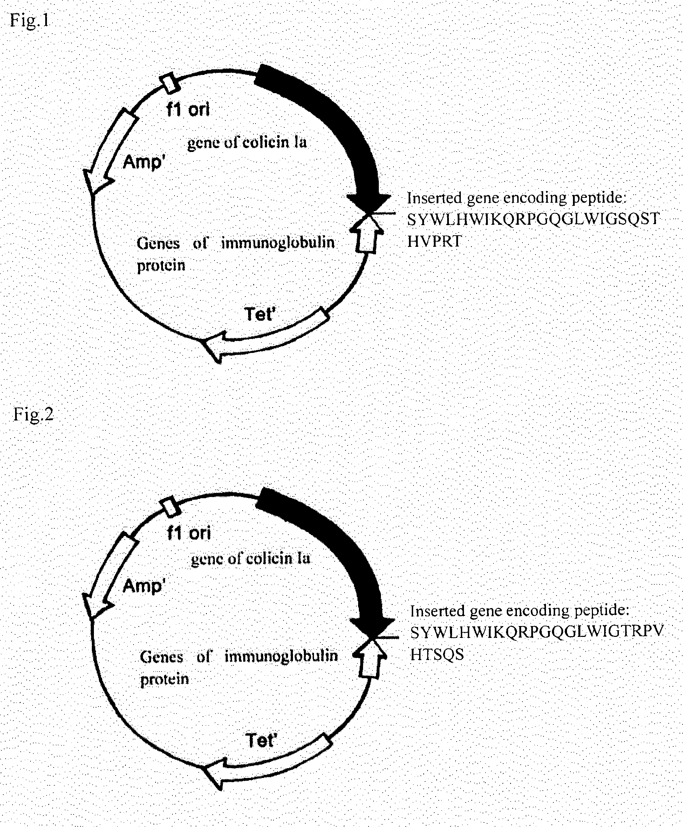

The original plasmid was pSELECT^{™}-1 plasmid (8.3kb) with genes of Colicin Ia and immunity protein. By Double Strands Oligo nucleotide Point Mutation Technology (QuickChange^{™} Kit, Strategene corporation) the gene encoding the antibody mimetic set forth in SEQ ID NO. 1 or 3 were inserted to 626 amino acid position of Colicin la gene. Two recombinant plasmids, herein referred to as pBHC-PorA 1 and pBHC-PorA2 shown in Fig.1 and Fig. 2, were constructed, which were used to prepare the novel antibiotics. The recombinant plasmids were respectively transfected into *E.coli* BL-21 engineering bacterium to express the novel antibiotics.

The mutation procedure was preceded according to the manual of Strategene Quick Change Site Directed Mutagenesis Kit (catalog #200518),
1. Point mutation reactant was prepared as follows:
   5µl 10X buffer
   2µl (10ng) original plasmid pSELECT^{™}-1 with genes of Colicin Ia and immunity protein.
   1.25µl (125ng) artificial 5'-3' oligo nucleotide primer (refer to below oligo nucleotide primer)
   1.25µl (125ng) artificial 3'-5' oligo nucleotide primer (refer to below oligo nucleotide primer)
   1µl dNTP
   50µl de-ionized water
   1µl Pfu
      (The above drugs were all reagents in the medical kit, except plasmid, primer and de-ionized water.)
2. PCR amplification was proceeded with the amplification conditions as follow: denaturalize at 95 °C for 35 seconds, anneal at 53°C for 70 seconds, extend at 68°C for 17 minutes, totally 20 cycles.
3. 1µl endonuclease Dpn1 was incorporated to digest parent DNA chain(37°C, 1h); 1µl digestion product was placed on ice and incubated with 50µl XL1-Blue competent cells for 30 minutes, heat shock at 42 °C for 45 seconds, and then taken into ice for 2 minutes;
4. 0.5ml NZY culture medium was added, the bacteria solution (reactant of step 3, i.e. transformed cells from competent cells) was shaken at 220 rpm and 37°C for 1 hour; then 50-100 µl reactant was taken out to plank on medium plate (LB culture medium with 1% agar and 50µg/ml ampicillin, at 37°C over night);
5. The bacteria was picked out after cultivating 18 hours, the plasmid was abstracted and sequenced to ascertain it had successful mutation;
6. 100ng mutated plasmid was placed on ice and incubated with 40 µl of BL-21 competent cells for 5.minutes, heat shock at 42°C for 30 seconds, and then placed on ice for 2 minutes. 160 µl SOC culture medium was added; bacteria was shaken at 220 rpm, 37°C for 1 hour and taken out to plank on medium plate (LB culture medium with 1% agar, 50µg/ml ampicillin, at 37°C cultivating one night); monoclone colonies are picked out for largely reproducing;
7. The bacteria was largely reproduced in 8-10L FB culture medium, at 250 rpm, 30°C for 3-4 hours, and warmed to 42°C at 250 rpm for 0.5hours and then cooled to 37°Cat 250 rpm for 1.5hours. The thallus was centrifugated at 4°C, 6000g for 20 minutes, and then was suspended in 80-100 ml of 50mM boric acid buffer fluid (pH 9.0, with 2mM EDTA) at 4°C. After being added in 50µg PMSF the thallus was ultrasonicated at 4°C, 400W for 1 minute and repeated 4-5 times with 2-3 minutes interval for maintaining the temperature of the bacteria solution. The cracked thallus was high-speed centrifugated at 4°C, 75,000g for 90 minutes. The 5,000,000 unit streptomycin sulfate was added into the supernatant to deposit DNA (stiring at 4 °C for 1 hour). After centrifugated at 10000g, 4°C for 10 minutes, the supernatant was loaded in bag filter of 15,000 molecular weight and dialysed by 10L of 50 mM boric acid buffer fluid over night at 4°C; then centrifugated again at 10000g, 4°C for 10 minutes. The supernatant was loaded on CM ion-exchange column. Then the CM ion-exchange column was washed thoroughly and the novel antibiotic was eluted by 0.3 M NaCl+50 mM boric acid buffer fluid. Corresponding to the above two kinds of recombinant plasmid, the novel antibiotics were named PMC-AM1 and PMC-AM2 of which the amino acid sequences were set forth in Seq ID No.6 and Seq ID No.8 respectively.

The AM1 was a peptide chain comprising of the peptides of the first complementarity determining domain in variable region of the heavy chain, the peptide of the second frame region of the heavy chain and the peptide of the third complementarity determining domain in variable region of the light chain. Its amino acid sequence was set forth in Seq ID No.2. The AM2 was a peptide chain comprising of the peptide of the first complementarity determining domain in variable region of the heavy chain, the peptide of the second frame region of the heavy chain and the peptide of reversed third complementarity determining domain in variable region of the light chain. Its amino acid sequence was set forth in Seq ID No.4.

The PMC-AM2 was constructed as a control of the PMC-AM I for testing the activity of the novel antibiotics of present invention when the domains composing the antibody mimetic were connected in different order.

The artificial oligo nucleotide sequences for preparing above two mutation plasmids respectively are as follow:
5'-3' (SEQ ID NO.9)
   gcg aat aag ttc tgg ggt att *TCT TAT TGG CTG CAT TGG ATT AAA CAG* taa ata aaa tat aag aca ggc
3' -5' (SEQ ID NO.10)
   gcc tgt ctt ata ttt tat tta *CTG TTT AAT CCA ATG CAG CCA ATA AGA* aat acc cca gaa ctt att cgc
5'-3' (SEQ ID NO.11)
3'-5' (SEQ ID NO.12)
   gec tgt ctt ata ttt tat tta *TCC GAT CCA CAG TCC CTG ACC AGG TCT* ctg ttt aat cca atg cag cca
5' - 3' (SEQ ID NO.13)
3'- 5' (SEQ ID NO.14)
   gcc tgt ctt ata ttt tat tta *GGT TCT CGG CAC ATG CGT GGA CTG AGA* tcc gat cca cag tcc ctg acc
   pBHC-PorA 2
5'-3' (SEQ ID NO.15)
   gcg aat aag ttc tgg ggt att *TCT TAT TGG CTG CAT TGG ATT AAA CAG* taa ata aaa tat aag aca ggc
3'-5' (SEQ ID NO.16)
   gcc tgt ctt ata ttt tat tta *CTG TTT AAT CCA ATG CAG CCA ATA AGA* aat acc cca gaa ctt att cgc
5'-3' (SEQ ID NO.17)
3'-5' (SEQ ID NO. 18)
   gcc tgt ctt ata ttt tat tta *TCC GAT CCA CAG TCC CTG ACC AGG TCT* ctg ttt aat cca atg cag cca
5'-3' (SEQ ID NO.19)
3' - 5' (SEQ ID NO.20)
   gcc tgt ctt ata ttt tat tta *AGA CTG GGA CGT ATG CAC CGG TCT GGT* tcc gat cca cag tcc ctg acc.

### Embodiment 2: Inhibiting effect of the novel antibiotic on Nesseria meningitidis.

The bacteria was strain No. 29332 *Nesseria meningitidis* of Bacteria Preservation Center in China), two microliters (µl) of bacteria solution (10⁵CFU/ml) was added in 10ml rabbit blood-chocolate medium (containing 50mg beef extract, 100mg tryptone, 50mg NaCl, 30 mg K₂HPO₄ and 0.5-0.8 ml off fiber rabbit blood). Five groups were prepared. The first group as control was added 0.3 M NaCl + 50 mM boric acid buffer fluid (i.e. blank preservative fluid for the novel antibiotic, by the same volume as the novel antibiotic solution in the experimental groups). Penicillin sodium was added in the second group by 5µg/ml. The novel antibiotic PMC-AM1 was added in the third group by 5µg/ml. The novel antibiotic PMC-AM2 was added in the forth group by 5µg/ml. The novel antibiotic PMC-AM 1 was added in the fifth group by 10µg/ml.

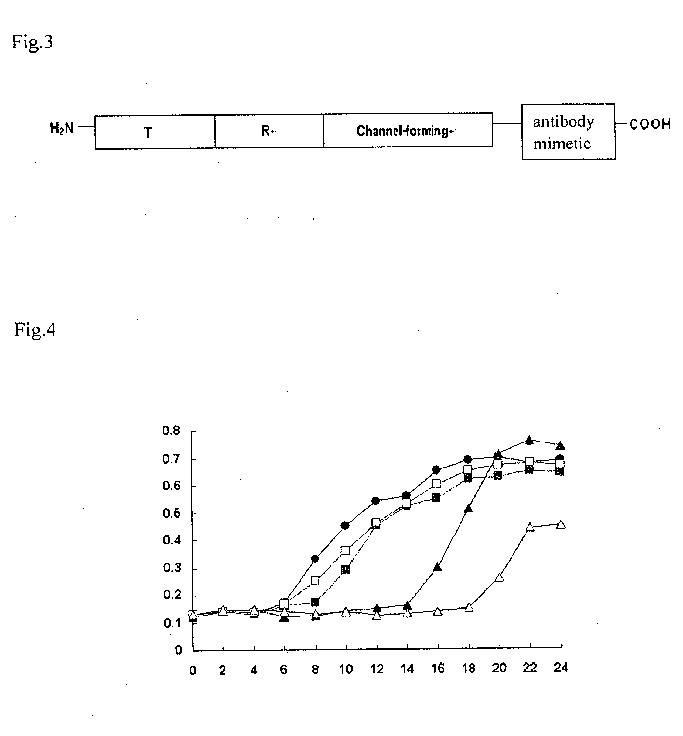

Reactant liquid of the above five groups were respectively put into 100ml conical flask, and cultured at 37°C by 200rpm. 100µl culture solution was sampled per hour and added onto 96-pore ELISA plate for measuring bacterial turbidity by spectrophotometer (A595nm) color comparison. The bacteria-growth curve was drawn up to compare the bacteriostatic efficacy of the novel antibiosis. The result, as shown in Fig.4, showed that *Nesseria meningitidis* could only be restrained by PMC-AM1.

### Embodiment 3. Contrast experiment of the minimum inhibitory concentration of the novel antibiotic and normal antibiotics on multi-drug resistant Pseudomonas aeruginosa.

Testing the minimum inhibitory concentration (MIC) of the novel antibiotic by the agar dilution method. The bacteria were inoculated on the surface of agar plate containing different concentrations of drugs by multipoint inoculate instrument (Deneley A400). The bacteria concentration on per point was 10⁵ CFU/ml. After incubated at 37°C for 18-24 hours, the result can be observed. The least concentration of drugs in the plating medium without bacteria growth was Minimum Inhibitory Concentration (MIC) of the drug to the said bacteria.

Experimental strain was multi-drug resistant *Pseudomonas aeruginosa* which was a clinical isolated strain (isolated by West China Hospital, No.13578) using MH medium (per 100ml containing 500mg beef extract, 1.75g casein acid hydrolysate, 150mg soluble starch and 1.7g agar) .

As the result showed in Fig.5, the MIC of the novel antibiotic (D) PMC-AM1 on multi-drug resistant *Pseudomonas aeruginosa* was 8 µg/ml, ceftazidime (A) was 16 µg/ml, levofloxacin (B) was 8 µg/ml, and gentamicin (C) was greater than 512 µg/ml. If in terms of molecular weight standard, the MIC of PMC-AM 1 on multi-drug resistant *Pseudomonas aeruginosa* was 0.23 nMol, ceftazidime (A) was 29.3 nMol, levofloxacin was 43.2 nMol, and gentamicin(C) was greater than 890 nMol, i.e. the antibacterial effect of PMC-AM1 on multi-drug resistant *Pseudnmonas aeruginosa* was stronger 127-3800 times than ceftazidime, levofloxacin and gentamicin.

### Embodiment 4. Contrast experiment of the antibacterial activity in vitro between the novel antibiotic of this invention and normal antibiotics.

Testing the minimum inhibitory concentration (MIC) of the novel antibiotic by the agar dilution method. The bacteria were inoculated on the surface of agar plate containing different concentrations of drugs by multipoint inoculate instrument (Deneley A400). The bacteria concentration per point was 10⁵CFU/ml. After incubated at 37°C for 18-24 hours, the result can be observed. The least concentration of drugs in the plating medium without bacteria growth was Minimum Inhibitory Concentration (MIC) of the drug to the said bacteria.

Experimental strains were multi-drug resistant *Pseudomonas aeruginosa* which is a clinical isolated strain (isolated by West China Hospital, No.13578) using MH medium (Per 100ml containing 500mg beef extract and 1.75g_casein acid hydrolyzate, 150mg soluble starch, 1.7g_gelose), methicillin-resistant *Staphylococcus aureus* (ATCC BAA-42) using BM medium (Per 100ml containing 1g tryptone, 0.5g yeast powder, 0.1g glucose, 100mg KH₂PO₄, 1g NaCL and 1g_agar), vancomycin-resistant *Enterococcus faecalis* (ATCC 700802) using the MH medium; *Nesseria meningitidis* (No.29332 of Bacteria Preservation Center in China) using the same medium used in the_embodiment 2 (in addition added Columbia blood agar base 3.9g).

The result shown in Fig.6, A showed the result of *Nesseria meningitidis:* (1) PMC-AM1, MIC = 0.11nMol, (2) ceftazidime, MIC=3.02 nMol, (3) ampicillin, MIC =1.35 nMol. B showed the result of vancomycin-resistant *Enterococcus faecalis:* (1) PMC-AM1, MIC=0.23 nMol, (2) vancomycin, MIC=21.54 nMol, (3) ampicillin, MIC=10.78 nMol. C showed the result for methicillin-resistant *Staphylococcus aureus:* (1) PMC-AM1, MIC=0.06nMol, (2) ampicillin MIC=21.55nMol, (3) oxacillin, MIC =14.1 nMol. D showed the result for multi-drug resistant *Pseudomonas aeruginosa*: (1) PMC-AM1, MIC=0.91nMol, (2) levofloxacin, MIC=43.2 nMol, (3) ceftazidime, MIC=29.3 nMol (4) gentamicin, MIC>889.4 nMol.

### Embodiment 5. Contrast experiments of the antibacterial activity in vitro between the novel antibiotic of this invention, the polypeptide anti-Staphylococcal aureus and wild-type Colicin Ia.

Testing the minimum inhibitory concentration (MIC) of the novel antibiotic by the agar dilution method. The least concentration of drugs in the plating medium without bacteria growth was Minimum Inhibitory Concentration (MIC) of the drug to the said bacteria.

Experimental strains were multi-drug resistant *Pseudomonas aeruginosa* (isolated by West China Hospital, No.13578),methicillin-resistant *Staphylococcus aureus* (ATCC BAA-42), vancomycin-resistant *Enterococcus faecalis* (ATCC 700802), using MH medium ; *Nesseria meningitidis* (No.29332 of bacteria Preservation Center in China).

The results shown in Fig.7, A showed the result for vancomycin-resistant *Enterococcus faecalis* : (1) the polypeptide anti*-Staphylococcal aureus,* MIC = 0.91 nMol, (2) the wildtype Colicin Ia, MIC =0.91 nmol, (3) PMC-AM1,MIC = 0.23 nmol. B showed the result for methicillin-resistant *Staphylococcus aureus:* (1) the polypeptide anti*-Staphylococcal aureus,* MIC = 0.06nMol, (2) the wildtype Colicin Ia, MIC=0.23 nMol, (3) PMC-AM1, MIC=0.06nMol. C showed the result for multidrug resistance *Pseudomonas aeruginosa*:(1) the polypeptide anti-*Staphylococcal aureus,* MIC = 0.91nMol, (2) the wildtype Colicin Ia, MIC =0.91 nMol, (3) PMC-AM1, MIC = 0.23 nMol

### Embodiment 6. In vivo protection experiments of the novel antibiotics for animals infected by Nesseria meningitidis.

### Experimental materials

Drugs: PMC-AM1, gentamicin, ampicillin.

### Experimental bacteria

*Nesseria meningitides* (No.29332 of Bacteria Preservation Center in China, i.e Center of Medical Devices of National Institute for the Control of Pharmaceutical & biological Products, SDA).

### Experimental methods

As shown in Fig.7, 40 mice were divided into four experimental groups, 10 mice in each group. The mice were given an intraperitoneal injection of glucose solution of ferrous by 20mg/kg, and 1 hour later an intraperitoneal injection of 0.5 ml of bacteria culture containing 1 share of *Nesseria meningitidis*cultrue solution (its CFU was 2.36 x 10⁹/ml) and 1.5 share of 5% dry yeast solution. One hour later after being given intraperitoneal injection of fatal dose of the bacteria culture, the mice in experimental group were given an intravenous injection of the drugs and the mice in control group were given an intravenous injection of normal saline, all drugs were injected by 1.5mg/kg), observation every 2 hours for 8 days, the death of mice as the positive results.
In the Fig.7, 1.PMC-AM1 means the novel antibiotic obtained in this invention; 2. Gen means gentamicin; 3. PEN means penicillin; 4. Con. means control.

### Results.

As the survive curve shown in Fig.7, after being given intraperitoneal injection of fatal dose of *Neisseria meningitides* solution, 1). Mice in the control group all died in two days, 2). Mice in penicillin group all died in two days, 3). Mice in gentamicin group had 50%survival rate in 8 days, 4).Mice in PMC-AM1 group had 90% survival rate in 8 days.

The result indicates the novel antibiotic PMC-AM 1 of the present invention performed superior protection activity in vivo on mice infected by fatal dose of *Nesseria meningitidis* than common antibiotics.

## Claims

1. An antibiotic comprising an antibody mimetic and a colicin, or comprising an antibody mimetic and a channel-forming domain of a colicin, the antibody mimetic covalently bonded to the carboxyl end of the polypeptide of the colicin or the channel-forming domain of the colicin, wherein the colicin is selected from the group consisting of colicin E1, Ia, Ib, A, B, N; wherein said antibody mimetic being yielded by fusing two complementarity determining regions (CDRs), V_{H}CDR₁, and V_{L}CDR₃ through a cognate framework region V_{H}FR₂ of an immunoglobulin; wherein the antibody mimetic specifically recognizes bacterium porins.

2. The antibiotic of claim 1, wherein the bacterium is *Neisseria meningitides,* the porin is PorA in the outer membrane of *Neisseria meningitidis* cells.

3. The antibiotic of claim 2, wherein the immunoglobulin consist of covalent bonded polypeptide of SEQ ID NO: 21 and a polypeptide of SEQ ID NO: 22.

4. The antibiotic of claim 1 or claim 2 or claim 3, wherein the colicin is Ia.

5. A peptide molecule comprising an antibody mimetic and a colicin, or comprising an antibody mimetic and a channel-forming domain of a colicin, the antibody mimetic covalently bonded to the carboxyl end of the polypeptide of the colicin or the channel-forming domain of the colicin, wherein the colicin is selected from the group consisting of colicin E1, Ia, Ib, A, B, N; wherein said antibody mimetic being yielded by fusing two complementarity determining regions (CDRs), V_{H}CDR₁, and V_{L}CDR₃ through a cognate framework region (V_{H}FR₂) of an immunoglobulin; wherein the immunoglobulin specifically recognizes bacterium porins.

6. One of the fusion peptide molecules of claim 5, its amino acid sequence sets forth in SEQ ID NO.6.

7. A nucleic acid molecule encoding a fusion peptide molecule claimed in claim 5 or claim 6.

8. The nucleic acid molecule of claim 7, its nucleotide sequence sets forth in SEQ ID NO.5.

9. A recombinant plasmid containing a nucleic acid molecule of claims 7 or 8.

10. A preparation method of any one of the antibiotics claimed in claims 1-4, one of the recombinant plasmids of claim 9 is transfected into an expression system, and the antibiotic is separated and purified from expressed polypeptide.

11. The antibiotic of any one of claims 1-4 for use in preparing an antibacterial medicament.

12. The antibiotic for use according to claim 11, wherein the antibacterial medicament is used for killing *Neisseria meningitidis,* vancomycin-resistant *Enterococcus faecalis,* methicillin-*resistant Staphylococcus aureus or multidrug-resistance Pseudomonas aeruginosa.*

## Patentansprüche

1. Ein Antibiotikum, das eine Antikörpernachahmung und ein Colicin umfasst, oder eine Antikörpernachahmung und eine kanalbildende Domäne eines Colicins umfasst, wobei die Antikörpernachahmung kovalent an das Carboxylende des Polypeptids des Colicins oder die kanalbildende Domäne des Colicins gebunden ist, wobei das Colicin ausgewählt ist aus der Gruppe bestehend aus Colicin E1, la, Ib, A, B, N; wobei besagte Antikörpernachahmung erhalten wird durch Fusion von zwei Antigenbindungsstellen (CDRₛ), V_{H}CDR₁ und V_{L}CDR₃ durch eine verwandte Rahmenregion V_{H}FR₂ eines Immunoglobulins, wobei die Antikörpernachahmung spezifisch an Bakterium-Purine bindet.

2. Das Antibiotikum nach Anspruch 1, wobei das Bakterium *Neisseria meningitides* ist, das Purin PorA in der äußeren Membran von *Neisseria meningitidis*-Zellen ist.

3. Das Antibiotikum von Anspruch 2, wobei das Immunglobulin aus kovalent gebundenem Polypeptid von SEQ ID NO: 21 und einem Polypeptid von SEQ ID NO: 22 besteht.

4. Das Antibiotikum von Anspruch 1 oder 2 oder Anspruch 3, wobei das Colicin la ist.

5. Ein Peptidmolekül umfassend eine Antikörpernachahmung und ein Colicin, oder umfassend eine Antikörpernachahmung und eine kanalbildende Domäne eines Colicins, wobei die Antikörpernachahmung kovalent an das Carboxylende des Polypeptids des Colicins, oder der kanalbildenden Domäne des Colicins gebunden ist, wobei das Colicin ausgewählt ist aus der Gruppe bestehend aus Colicin E1, la, Ib, A, B, N; wobei besagte Antikörpernachahmung erhalten wird durch Fusion von zwei Antigenbindungsstellen (CDRₛ), V_{H}CDR₁, und V_{L}CDR₃ durch eine verwandte Rahmenregion (V_{H}FR₂) eines Immunoglobulins; wobei das Immunoglobulin spezifisch an Bakterium-Purine bindet.

6. Eines der Fusions-Peptid-Moleküle von Anspruch 5, dessen Aminosäuresequenz in SEQ ID NO: 6 dargestellt ist.

7. Ein Nukleinsäuremolekül, das ein Fusionspeptidmolekül beansprucht in Ansprüchen 5 oder 6 kodiert.

8. Das Nukleinsäuremolekül von Anspruch 7, dessen Nukleotidsequenz dargestellt ist in SEQ ID NO: 5.

9. Ein rekombinantes Plasmid, das ein Nukleinsäuremolekül von Ansprüchen 7 oder 8 umfasst.

10. Ein Herstellungsverfahren von irgendeinem der Antibiotika, beansprucht in den Ansprüchen 1 bis 4, wobei eines der rekombinanten Plasmide von Anspruch 9 transfiziert wird in ein Expressionssystem und das Antibiotikum abgetrennt und aufgereinigt wird aus dem exprimierten Polypeptid.

11. Das Antibiotikum von irgendeinem der Ansprüche 1 bis 4 zur Verwendung beim Herstellen eines antibakteriellen Medikaments.

12. Das Antibiotikum zur Anwendung gemäß Anspruch 11, wobei das antibakterielle Medikament verwendet wird zum Abtöten von *Neisseria meningitidis*, Vancomycinresistentem *Enterococcus faecalis,* Methicillin-resistentem *Stephylococcus aureus* oder multiresistentem *Pseudomonas aeruginosa.*

## Revendications

1. Un antibiotique comprenant un mimétique d'anticorps et une colicine, ou comprenant un mimétique d'anticorps et un domaine de formation de canal d'une colicine, le mimétique d'anticorps lié de façon covalente à une extrémité carboxyle du polypeptide de la colicine ou au domaine de formation de canal de la colicine, dans lequel la colicine est sélectionnée parmi le groupe consistant en la colicine E1, la, Ib, A, B, N; dans lequel ledit mimétique d'anticorps étant produit par fusion de deux régions déterminantes complémentaires (CDRs), V_{H}CDR₁, et V_{L}CDR₃ à travers une région d'ossature apparenté V_{H}FR₂ d'une immunoglobuline; dans lequel le mimétique d'anticorps reconnaît spécifiquement des porines bactériennes.

2. L'antibiotique selon la revendication 1, dans lequel la bactérie est une *Neisseria meningitides,* la porine est une PorA dans la membrane externe des cellules *Neisseria meningitidis*.

3. L'antibiotique selon la revendication 2, dans lequel l'immunoglobuline consiste en un polypeptide de SEQ ID NO: 21 lié de façon covalente et un polypeptide de SEQ ID NO: 22.

4. L'antibiotique selon la revendication 1 ou la revendication 2 ou la revendication 3, dans lequel la colicine est la.

5. Une molécule peptidique comprenant un mimétique d'anticorps et une colicine, ou comprenant un mimétique d'anticorps et un domaine de formation de canal d'une colicine, le mimétique d'anticorps lié de façon covalente à une extrémité carboxyle du polypeptide de la colicine ou au domaine de formation de canal de la colicine, dans laquelle la colicine est sélectionnée parmi le groupe consistant en la colicine E1, la, Ib, A, B, N; dans laquelle ledit mimétique d'anticorps étant produit par fusion de deux régions déterminantes complémentaires (CDRs), V_{H}CDR₁, et V_{L}CDR₃ à travers une région d'ossature apparenté (V_{H}FR₂) d'une immunoglobuline; dans laquelle l'immunoglobuline reconnaît spécifiquement des porines bactériennes.

6. Une des molécules peptidiques de fusion selon la revendication 5, sa séquence d'acides aminés est telle que décrite dans SEQ ID NO.6.

7. Une molécule d'acide nucléique codant pour une molécule peptidique de fusion revendiquée dans la revendication 5 ou la revendication 6.

8. La molécule d'acide nucléique selon la revendication 7, sa séquence de nucléotides est telle que décrite dans SEQ ID NO.5.

9. Un plasmide recombinant contenant une molécule d'acide nucléique selon la revendication 7 ou 8.

10. Un procédé de préparation d'un quelconque des antibiotiques revendiqués dans les revendications 1 à 4, un des plasmides recombinants selon la revendication 9 est transfecté au sein d'un système d'expression et l'antibiotique est séparé et purifié à partir du polypeptide exprimé.

11. L'antibiotique selon une quelconque des revendications 1 à 4 pour utiliser dans la préparation d'un médicament antibactérien.

12. L'antibiotique pour utiliser selon la revendication 11, dans lequel le médicament antibactérien est utilisé pour tuer la *Neisseria meningitidis, Enterococcus faecalis* résistant à la vancomycine, *Staphylococcus aureus* résistant à la méthicilline ou *Pseudomonas aeruginosa* résistant à plusieurs médicaments.
